# EUROPEAN PATENT APPLICATION

(11) **EP 3 719 112 A1**
(43) Date of publication of application: **07.10.2020**
(21) Application number: 18883264.6
(22) Date of filing: 18.10.2018
(51) Int. Cl.: C12M 3/00, A61K 35/12, A61L 27/16, A61L 27/56, A61P 43/00, C12N 5/071

(54) **IMMUNOISOLATION DEVICE**

(30) Priority: 30.11.2017 JP 2017230247
(71) Applicant: Hitachi, Ltd., Tokyo 100-8280 (JP)
(72) Inventor: MARUYAMA Masashi, Tokyo 100-8280 (JP); MATSUMORI Masaki, Tokyo 100-8280 (JP)
(74) Representative: MERH-IP Matias Erny Reichl Hoffmann Patentanwälte PartG mbB
(86) International application number: PCT/JP2018/038760
(87) International publication number: WO 2019/106996

(57) **Abstract**

The purpose of the present invention is to solve conventional problems involving immunoisolation devices using a porous membrane as an immunoisolation membrane, such problems including fibrotic formation on the device surface, shortage of oxygen supplied to cells, and non-uniform distribution of cells. An immunoisolation device 1 for transplantation is characterized by comprising: an immunoisolation membrane 10; and an oxygen supply mechanism 20 and a three-dimensional cell support 30 which are tightly enclosed inside the immunoisolation membrane 10 by physical sealing, a biocompatible adhesive, or a combination thereof, wherein the immunoisolation membrane 10 is a porous membrane provided with a hydrophilic layer on the outer surface, and the oxygen supply mechanism 20 can supply oxygen to cells 40 supported by the three-dimensional cell support 30 through an oxygen permeable membrane 21.

## Description

### Technical Field

The present invention relates to an immunoisolation device.

### Background Art

In regenerative medicine by transplantation of cells of the endocrine system or cells exhibiting a paracrine effect, suppression of attack of therapeutic cells by immune cells and low cell fixation rate are problems. In addition, when using cells derived from iPS cells, the risk of canceration is a problem. Against such a background, a method of transplanting cells encapsulated using a porous membrane as an immunoisolation membrane has been developed (NPL 1). For example, a method of introducing a cell suspension into a bag-like immunoisolation device made of a porous membrane (PTL 1) has been known, but there are problems that the device surface tends to fibrillate, it is difficult to supply oxygen to cells, distribution of cells tends to be biased, and the like. As a method for supplying oxygen into the device, a method using an electrochemical reaction in the body (PTL 2) has been known, but there are problems that the volume of the device to be transplanted becomes large, and the like. Further, a method of supplying gaseous oxygen into the device through an oxygen permeable membrane (PTL 3) and a method of supplying oxygen by containing an oxygen sustained release material in an oxygen permeable material (PTL 4) have been known, but it is not easy to achieve compatibility with other issues.

### Citation List

### Non-Patent Literature

NPL 1: Rev. Diabet. Stud. 2014, 11, 84-101.

### Patent Literature

PTL 1: US 8,278,106
PTL 2: US 2003/0,087,427 A
PTL 3: US 8,784,389
PTL 4: US 2012/0,114,729 A

### Summary of Invention

### Technical Problem

Therefore, an object of the present invention is to solve the conventional problems of fibrosis on the device surface, insufficient supply of oxygen to cells, and bias of cells, in an immunoisolation device using a porous membrane as an immunoisolation membrane.

### Solution to Problem

In order to solve the problems, the present invention is an immunoisolation device for transplantation including an immunoisolation membrane, and an oxygen supply mechanism and a three-dimensional cell support which are tightly enclosed inside the immunoisolation membrane by physical sealing, a biocompatible adhesive, or a combination thereof, in which the immunoisolation membrane is a porous membrane provided with a hydrophilic layer on the outer surface, and the oxygen supply mechanism can supply oxygen to cells supported by the three-dimensional cell support through an oxygen permeable membrane.

This description includes the disclosure of Japanese Patent Application No. 2017-230247, which is a priority document of the present application.

### Advantageous Effects of Invention

According to the present invention, in an immunoisolation device, it is possible to suppress fibrosis on the device surface, improve cell viability by supplying oxygen, and uniformize oxygen supply to cells and uniformize expression of cellular functions by suppressing cell bias. Also, problem, constitution and effect other than those described above will be revealed from the description of the following embodiments.

### Brief Description of Drawings

[FIG. 1] FIG. 1 is a schematic diagram of an immunoisolation device according to an embodiment of the present invention.
[FIG. 2] FIG. 2 is a schematic diagram of an immunoisolation membrane.
[FIG. 3] FIG. 3 is a schematic diagram of an oxygen supply mechanism in an embodiment of the present invention.
[FIG. 4] FIG. 4 is a schematic diagram showing a production process of an immunoisolation device using a bead-like three-dimensional cell support.
[FIG. 5] FIG. 5 is a schematic diagram showing a production process of an immunoisolation device using a sponge-like three-dimensional cell support.
[FIG. 6] FIG. 6 is a schematic diagram for describing a synergistic effect obtained by using an oxygen supply mechanism capable of changing volume and a three-dimensional cell support in combination.
[FIG. 7] FIG. 7 is a schematic diagram showing a method for exchanging a solution in a cell-containing immunoisolation device.

### Description of Embodiments

Hereinafter, embodiments of the present invention will be described with reference to the drawings and the like. Since the following embodiments are to show specific embodiments of the present invention, the present invention is not to be considered limited to these embodiments, and various alterations and modifications can be made by those skilled in the art within the scope of the technical idea as disclosed in the present specification. In addition, in all drawings for explaining the present invention, like parts having like functions are designated by like reference numerals without repeating the description thereof.

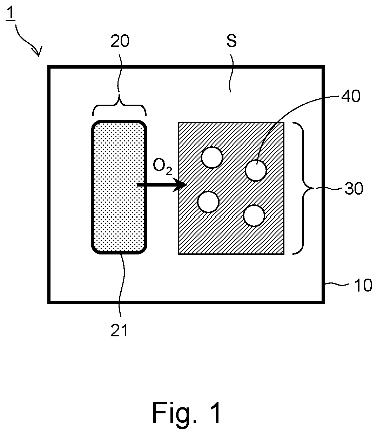

As shown in FIG. 1, an immunoisolation device 1 according to the present embodiment is intended for transplantation, and includes an immunoisolation membrane 10, an oxygen supply mechanism 20 and a three-dimensional cell support 30 which are tightly enclosed inside the immunoisolation membrane 10.

Cells 40 are introduced into the three-dimensional cell support 30 in the immunoisolation device 1 and the inside of the immunoisolation membrane 10 is filled with a solution S in which the cells can survive (hereinafter referred to as "cell-containing immunoisolation device") such as a medium can be transplanted into a non-human animal such as a mouse, a rat, a rabbit, a dog, a cat, a pig, a goat, a cow, a horse or a monkey, or a human. The oxygen supply mechanism 20 can supply oxygen to the cells 40 supported by the three-dimensional cell support 30 via an oxygen permeable membrane 21.

In the immunoisolation device 1, while the immunoisolation membrane 10 isolates transplanted cells from immune cells of a transplantation destination, needs not to inhibit take up of nutrients and humoral factors from body fluids and inflow of metabolites and humoral factors of the transplanted cells to the body fluids. In order to satisfy such requirements, a porous membrane having a pore diameter that allows a solute to pass through without allowing immune cells to pass through, provided with a hydrophilic layer on the outer surface, is used as the immunoisolation membrane 10. It is not always necessary to provide pores having a uniform pore diameter, which is only a guideline, but a desirable pore diameter is generally about 0.2 µm to 0.8 µm.

The material of the immunoisolation membrane 10 needs to be non-bioabsorbable and highly biocompatible. When a material having low biocompatibility is transplanted, inflammation and fibrosis progress due to a foreign body response, which is not desirable for treatment by cell transplantation. Examples of a porous membrane having an appropriate pore structure and expected to be biocompatible include fluororesin porous membranes such as porous PTFE (ePTFE) and electrospun PTFE.

However, fluororesins such as PTFE have high short-term biocompatibility, but not necessarily have high long-term biocompatibility. Therefore, it is preferable to form a hydrophilic layer on a fluororesin porous membrane with a hydrophilic compound having higher biocompatibility. Examples of a method for forming a hydrophilic layer include physical means for adsorbing or fixing a hydrophilic compound on the surface of a porous membrane without a covalent bond, and chemical means for introducing a hydrophilic compound via a covalent bond on the surface of a porous membrane to modify the surface. Such a hydrophilic layer may have not only an improvement in biocompatibility but also an action such as an increase in angiogenic action.

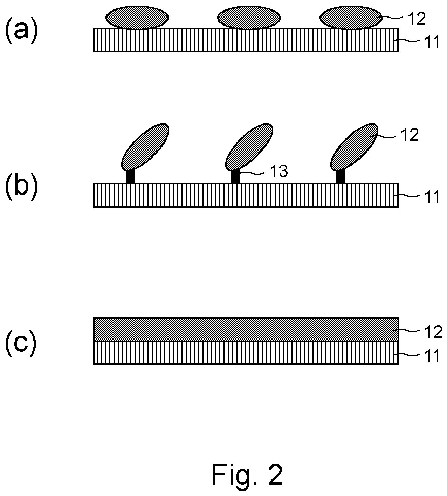

First, with respect to the physical means, a method of fixing the hydrophilic compound 12 on the surface of the porous membrane 11 by coating, as shown in FIG. 2(a), a method of laminating a layer containing the hydrophilic compound 12 on the surface of the porous membrane 11 by electrospinning or the like in which a laminate and hydrophilic fibers coexist, as shown in FIG. 2(c), and the like are considered. Examples of the hydrophilic compound for the hydrophilic layer thus formed include one or more selected from the group consisting of polyoxyethylene sorbitan fatty acid esters such as Tween 20 (trade name) and Tween 80 (trade name), collagen, gelatin, cellulose, agarose, alginic acid, chitosan, hydrophilic nylon, polyvinyl alcohol, polymethacrylic acid, polyethylene glycol (PEG), poly(2-methoxyethyl acrylate (PMEA) and 2-methacryloyloxyethyl phosphorylcholine polymers (PMPC) and derivatives thereof, but are not limited thereto. Examples of the derivatives include compounds obtained by modifying each of the above compounds by esterification, amidation or the like, compounds in which a part of the structure is substituted by hydrolysis, aminolysis or the like, copolymers containing each of the above compounds as a component, and the like.

On the other hand, with respect to the chemical means, as to fluororesins, surface treatment methods are very limited due to their chemical stability. In the method of creating reaction points on fluororesin by Na treatment, O₂ plasma treatment, sputtering treatment or the like, it may be unsuitable because the number of reaction points is small and surface modification efficiency is low, strength may be reduced, there is a risk that inflammation is likely to be induced on the contrary since the surface of the fluororesin gets rough, or the like. On the other hand, a method of creating radical reaction points on fluororesin by treatment with a low dose of electron beam or γ-ray is preferably used from the viewpoint of surface modification efficiency, strength, and surface roughness. In the surface treatment by chemical means, a hydrophilic compound (including a hydrophilic monomer and the like) is acted on a reaction point created by the treatment, whereby a surface on which the hydrophilic compound 12 is introduced via a covalent bond 13 can be obtained, as shown in FIG. 2(b). In addition to the reaction in one step, a method of introducing a precursor for a hydrophilic surface and then converting it to a hydrophilic compound, a method of introducing a polymer by acting another monomer after introducing an initiator or the like is applicable. Examples of the hydrophilic compound for the hydrophilic layer thus formed include one or more selected from the group consisting of polyethylene glycol (PEG), poly(2-methoxyethyl acrylate) (PMEA), 2-methacryloyloxyethyl phosphorylcholine polymers (PMPC), polyvinyl alcohol, polymethacrylic acid, polyacrylic acid, agarose, alginic acid, polypeptides and saccharides and derivatives thereof, but are not limited thereto. Examples of the derivatives include compounds obtained by modifying each of the above compounds by esterification, amidation or the like, compounds in which a part of the structure is substituted by hydrolysis, aminolysis or the like, copolymers containing each of the above compounds as a component, and the like.

The thinner the immunoisolation membrane 10, the more advantageous in terms of substance diffusion, but strength and reliability may be reduced. In order to solve this, the immunoisolation membrane 10 may be used together with a support material. The support material is a porous membrane or the like that has a large pore diameter and does not inhibit substance diffusion, but has sufficient strength to increase strength of the immunoisolation membrane 10, or the like. For example, a PTFE porous membrane having a pore diameter of 10 µm or more or the like corresponds to this.

By adsorbing a drug on the surface of the immunoisolation membrane 10, the drug can be slowly released after transplantation. Slow release of a drug having an antioxidant action, an anti-inflammatory action, an angiogenic action or the like is effective.

Fluororesin porous membranes such as PTFE having no hydrophilic layer have extremely high hydrophobicity, and thus pores are easily replaced by air in the air. However, when provided with a hydrophilic layer, water is easily retained in the pores even in the air, and can also be expected to have an effect of suppressing a risk of pore blocking before transplantation.

In the immunoisolation device 1 according to the present embodiment, the oxygen supply mechanism 20 is responsible for long-term oxygen supply to the cells 40. The oxygen supply mechanism 20 is particularly necessary when the immunoisolation device 1 into which cells have been introduced is transplanted to a site such as a subcutaneous site where blood vessels are small and oxygen partial pressure is low. The oxygen supply mechanism 20 supplies oxygen to the cells 40 by diffusing oxygen from an oxygen source having a high oxygen partial pressure into the immunoisolation device 1 having a low oxygen partial pressure via the oxygen permeable membrane 21.

As the oxygen source used by the oxygen supply mechanism 20, gaseous oxygen, an oxygen sustained-release material, an oxygen adsorbing material in which oxygen is previously adsorbed or the like can be used. The oxygen source may be used in the immunoisolation device 1 until exchange time, or the oxygen source may be supplied periodically from the outside.

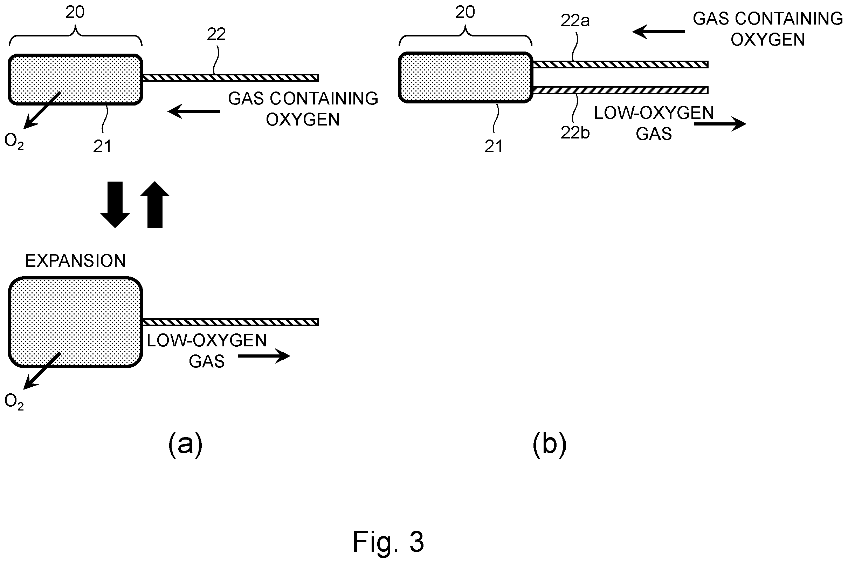

For example, in the case of gaseous oxygen, as shown in FIG. 3, a gas containing oxygen can be supplied from the outside of the immunoisolation device 1 to the inside of an oxygen supply mechanism including the oxygen permeable membrane 21 using a flow path. In this case, the oxygen permeable membrane 21 can be expanded (FIG. 3(a)) or not expanded (FIG. 3(b)) by the supply of gas from the outside. Further, as shown in FIG. 3(a), a single flow path 22 for supplying a gas containing oxygen is provided, and a low-oxygen gas generated after supplying oxygen via the oxygen permeable membrane 21 may be recovered via the same flow path 22, or the gas may be recovered through the flow path 22, or as shown in FIG. 3(b), two flow paths 22a and 22b are provided, and the supply of the gas containing oxygen and the recovery of the low-oxygen gas may be performed using each flow path.

From the viewpoint of oxygen permeability, the oxygen permeable membrane 21 is more advantageous as oxygen permeability coefficient is larger, thickness is smaller, and surface area is larger. Examples of a material having a large oxygen permeability coefficient include polydimethylsiloxane or a copolymer thereof, and the like. The thickness of the oxygen permeable membrane 21 is preferably about 10 µm to 1 mm from the viewpoint of transmission speed and reliability. In order to increase the strength of the oxygen permeable membrane 21, a porous support material or the like may be used in combination.

In the immunoisolation device 1 according to the present embodiment, the three-dimensional cell support 30 is used for controlling distribution of the cells 40.

When a conventional adhesive cell suspension or organoid suspension is introduced into a bag-like device, control of initial cell distribution, control of cell movement until the cells adhere, and control of movement of the adhered cells are difficult, and the distribution of cells tends to be biased. Further, in the case of non-adhesive cells, since the cells always float in the device, a bias occurs even after transplantation. As described above, unless special measures are taken, the cells tend to be biased, insufficient supply of nutrients and oxygen, non-uniform cell-cell interactions and the like occur, and a sufficient effect cannot be obtained. This problem becomes apparent particularly when the number of cells is large and the cells need to be filled at high density.

On the other hand, in the present embodiment, the three-dimensional cell support 30 is used for controlling the distribution of the cells 40. As a form of the three-dimensional cell support 30, a bead, fiber, mesh, nonwoven fabric or sponge form is applicable. By using such a three-dimensional cell support 30 having a high porosity, uniform distribution of the cells 40 and suppression of movement of the cells 40 in a flow of a liquid can be realized. Although a hydrogel can be used as the three-dimensional cell support 30, it is generally necessary to sufficiently increase the pore diameter because substance diffusion is slower as compared to that of the three-dimensional cell support 30 having a high porosity as described above.

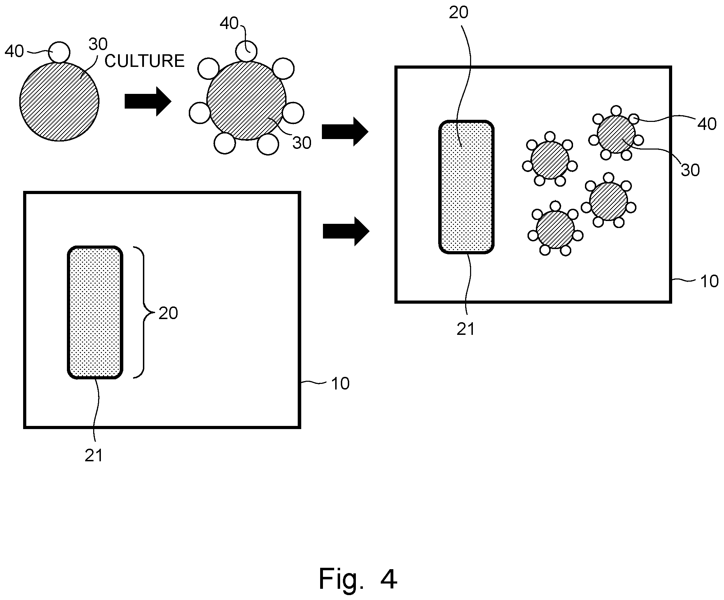

For example, as shown in FIG. 4, when using a bead-like three-dimensional cell support 30, the cells 40 are adhered to the bead-like three-dimensional cell support 30, and expansion culture, differentiation or maturation of the cells 40 or the like is performed by rotating culture using beads or the like as necessary, then the bead-like three-dimensional cell support 30 to which the cells 40 are adhered is recovered and introduced into the immunoisolation membrane 10 in which the oxygen supply mechanism 20 is tightly enclosed, whereby it is possible to easily and uniformly fill the cells 40 with high density.

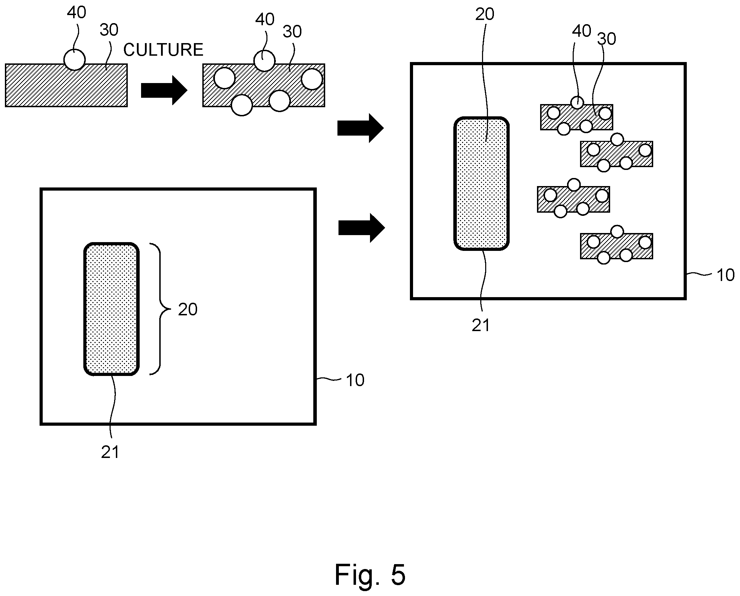

Further, for example, as shown in FIG. 5, when a mesh, nonwoven fabric or sponge-like three-dimensional cell support 30 is used, the cells 40 are uniformly seeded in a dish or flask provided with the three-dimensional cell support 30 and adhered, and expansion culture, differentiation or maturation of the cells 40 or the like is performed as necessary, then the three-dimensional cell support 30 to which the cells 40 are adhered is recovered and introduced into the immunoisolation membrane 10 in which the oxygen supply mechanism 20 is tightly enclosed, whereby it is possible to easily and uniformly fill the cells 40 with high density.

Cell adhesion to a fiber-like three-dimensional cell support and cell filling into a porous hollow fiber as a three-dimensional cell support are also effective in controlling cell distribution.

As shown in FIGS. 4 and 5, the cells 40 can be also adhered to the three-dimensional cell support 30 outside the immunoisolation membrane 10, and a cell-containing immunoisolation device can be also obtained by introducing a cell suspension into the immunoisolation membrane 10, with respect to a state where the three-dimensional cell support 30 that does not contain the cells 40 is previously tightly enclosed in the immunoisolation membrane 10.

Appropriate ranges of the porosity, specific surface area and the like of the three-dimensional cell support 30 to be used vary depending on the type and number of the target cells 40. In addition, the form and phenotype of the cell may be affected by the material, elastic modulus and curvature of the three-dimensional cell support 30, and the diameter, the type of surface functional group and the like when the three-dimensional cell support 30 is fibrous. Therefore, in order to obtain high effects, the form and properties of the three-dimensional cell support 30 are appropriately set.

The material of the three-dimensional cell support 30 is not particularly limited as long as it is a material that can support cells, but specific examples include one or more materials selected from the group consisting of polysaccharides such as cellulose, cross-linked agarose and chitosan, polypeptides such as cross-linked gelatin, resins such as polyethylene, polypropylene, polystyrene, polycarbonate, polyester, polysulfone and polylactic acid, silicone resins, polyisobutylene-containing rubber, soft resins like styrene-isobutylene block copolymer (SIB) and styrene-isobutylene-styrene block copolymer (SIBS), fluororesins such as polytetrafluoroethylene (PTFE), fluorine-based resins like polyvinylidene fluoride (PVDF), metal oxides like silica, metals like titanium, hydrogels like polyethylene glycol gels and derivatives thereof. Examples of the derivatives include those obtained by modifying each of the above materials by esterification, amidation or the like, materials in which a part of the structure is substituted by hydrolysis, aminolysis or the like, copolymers containing each of the above materials as a component, and the like. Further, the surface of the three-dimensional cell support 30 may be modified. The surface modification of the three-dimensional cell support 30 may be not only a simple hydrophilization or charge application but also a modification with an adhesion factor, a growth factor, an extracellular matrix (ECM), or the like. Although a biodegradable material such as polylactic acid can also be used, in this case, it is desirable to use under conditions in which decomposition of the three-dimensional cell support 30 and spontaneous formation of a scaffold by ECM secretion from the cells 40 proceed in parallel, or conditions in which the immunoisolation device itself is replaced before the three-dimensional cell support 30 is completely decomposed.

The three-dimensional cell support 30 may not be fixed in the immunoisolation device 1, but may be used in a state where the position is fixed or semi-fixed. Specifically, it may be fixation with an adhesive, a bolt, a pin or the like, or may be semi-fixation by putting the three-dimensional cell support 30 in a bag-like fixture. When a small and easily movable shape such as a bead or a small plate is used as the three-dimensional cell support 30, fixing or semi-fixing is particularly important for controlling the cell distribution and keeping the distance from the oxygen supply mechanism 20 constant. For example, a constant cell distribution can be maintained by putting the bead-like or plate-like three-dimensional cell support 30 into a flat bag-like fixture.

In the immunoisolation device according to the present embodiment, a synergistic effect can be also obtained by appropriately combining each component, for example, the oxygen supply mechanism 20 and the three-dimensional cell support 30.

For example, a three-dimensional structure can be formed on the surface of an oxygen permeable membrane provided in the oxygen supply mechanism using an oxygen permeable material, and this can be utilized as a three-dimensional cell support for cell adhesion. In this case, the oxygen-permeable three-dimensional structure not only contributes to the control of cell distribution as a three-dimensional cell support, but also functions as a part of a diffusion path of oxygen to the cells. Such oxygen supply via a three-dimensional cell support is one of the synergistic effects made possible by the combined use of the oxygen supply mechanism and the three-dimensional cell support.

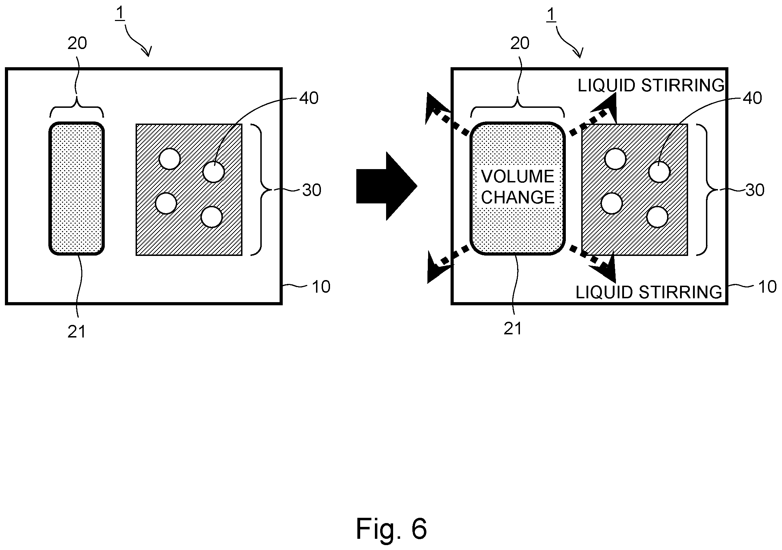

Further, for example, as shown in FIG. 6, when the oxygen supply mechanism 20 can be expanded by introducing a gas, volume change of the oxygen supply mechanism 20 due to introduction and exhaustion of the gas can be used for stirring a liquid in the immunoisolation device 1. When not provided with the three-dimensional cell support 30, such stirring of liquid has a high risk of inducing a bias in the distribution of the cells 40, but when provided with the three-dimensional cell support 30, it is possible to efficiently stir the liquid in the immunoisolation device 1 without exerting an adverse effect on the distribution of the cells 40. Such efficient oxygen supply by liquid stirring is one of the synergistic effects made possible by the combined use of the oxygen supply mechanism 20 and the three-dimensional cell support 30.

In the immunoisolation device 1 according to the present embodiment, the oxygen supply mechanism 20 and the three-dimensional cell support 30 are tightly enclosed inside the immunoisolation membrane 10 by physical sealing or a biocompatible adhesive or a combination thereof. Here, the physical sealing refers to, for example, sealing by heat fusion, folding, fitting of a frame, or the like. Also, the biocompatible adhesive refers to, for example, a biocompatible thermoplastic resin, an adhesive for medical equipment, an adhesive for surgery, a hemostatic agent, or the like. Examples of such a biocompatible adhesive include hemostatic materials like Hydrofit (registered trademark), fluorine-containing adhesives, polyethylene glycol-containing adhesives, cyanoacrylate-based adhesives like Histoacryl (registered trademark), or adhesives containing any one selected from the group consisting of polydimethylsiloxane (PDMS), styrene-isobutylene block copolymer (SIB), styrene-isobutylene-styrene block copolymer (SIBS), nylon, polyethylene terephthalate (PET), polypropylene (PP) and polycarbonate (PC) and derivatives thereof, and the like. Examples of the derivatives include those obtained by modifying each of the above adhesives by esterification, amidation or the like, adhesives in which a part of the structure is substituted by hydrolysis, aminolysis or the like, copolymers containing each of the above adhesives as a component, and the like. In the case of tightly enclosing by physical sealing such as thermal fusion, overall or local heating is required, and for heating, an oven, a heat sealer, an ultrasonic welding machine or the like can be used. Overall heating by an oven can also serve as dry heat sterilization depending on conditions.

The introduction of the cells 40 into the immunoisolation device 1 according to the present embodiment may be performed by introducing a cell suspension into the immunoisolation device 1 in a state where the three-dimensional cell support 30 is previously tightly enclosed, or may be performed by tightly enclosing the immunoisolation device 1 in a state where the cells 40 are supported by the three-dimensional cell support 30 inside the immunoisolation membrane 10. Also, in addition to the three-dimensional cell support 30, cells previously cultured in a high oxygen state with the whole or a part of the oxygen supply mechanism 20 may be tightly enclosed inside the immunoisolation membrane 10 to obtain a cell-containing immunoisolation device.

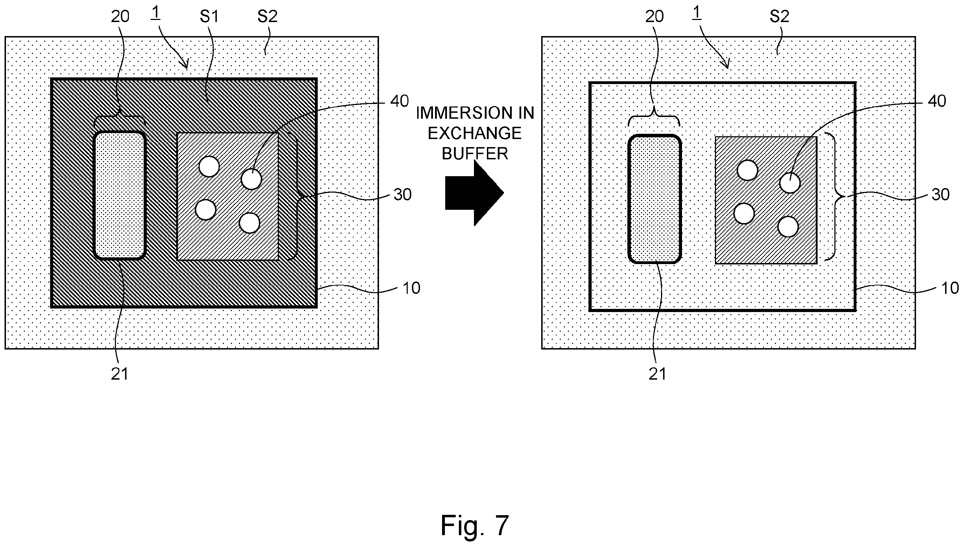

The cell-containing immunoisolation device according to the present embodiment can exchange a solution in the cell-containing immunoisolation device by being immersed in a solution different from the solution contained inside the immunoisolation membrane. That is, the immunoisolation device 1 has a high substance permeability due to the porous membrane, and since the cells 40 are supported by the three-dimensional cell support 30, buffer exchange with the cells 40 introduced is easy. In general, cells for transplantation require complicated operations such as centrifugation and dialysis for buffer exchange from a culture medium to a solution for transplantation. On the other hand, in the immunoisolation device 1 of the present embodiment, as shown in FIG. 7, since the buffer can be replaced without affecting the distribution of the cells 40 by immersing the immunoisolation device 1 in transplantation solution S2 after performing the introduction of the cells 40 in culture medium S1 as it is, operability is high. This is a synergistic effect obtained by combining the immunoisolation membrane 10 which is a porous membrane and the three-dimensional cell support 30, among the components of the immunoisolation device 1 according to the present embodiment.

The cell-containing immunoisolation device may be transplanted as it is, or may be put in some cover or the like and transplanted, then placed after removing the cover. In addition, the transplantation destination may be a human, a primate other than a human, a mammal in general, or an animal in general. The site to be transplanted may be subcutaneous, intraperitoneal or the like, but is not limited thereto. In addition, after transplantation, it may be removed after a certain period of time, or may not be removed.

### Examples

### (Example 1)

An immunoisolation device including a porous PTFE membrane including Tween 80 coating as a hydrophilic layer as an immunoisolation membrane, a cellulose nonwoven fabric as the three-dimensional cell support, and a PDMS tube filled with gaseous oxygen as the oxygen supply mechanism, and tightly enclosing a three-dimensional cell support and an oxygen supply mechanism was produced using Hydrofit (registered trademark) as a biocompatible adhesive.

Specifically, the PDMS tube wrapped with the cellulose nonwoven fabric was placed on the immunoisolation membrane, and the sides of the immunoisolation membrane were adhered using Hydrofit (registered trademark), so that the immunoisolation device in which the three-dimensional cell support and the oxygen supply mechanism were tightly enclosed inside was obtained.

### (Example 2)

An immunoisolation device including a porous PTFE membrane provided with a hydrophilic layer of PMEA graft as an immunoisolation membrane, a PDMS porous body on a thin plate coated with collagen as the three-dimensional cell support, and a PDMS bag capable of changing its volume by supplying air from the outside as the oxygen supply mechanism, and tightly enclosing a three-dimensional cell support and an oxygen supply mechanism was produced using SIBS as a biocompatible adhesive.

First, after forming a hydrophilic layer of PMEA graft on an immunoisolation membrane formed in a back shape by adhesion using SIBS, a PDMS porous body integrated with a PDMS bag was introduced, and finally, opening of the back-like immunoisolation membrane was folded and physically sealed, thereby obtaining an immunoisolation device in which the three-dimensional cell support and the oxygen supply mechanism were tightly enclosed inside.

### (Example 3)

An immunoisolation device including a porous PTFE membrane provided with a hydrophilic layer consisting of a laminate of a polymethacrylic acid-polymethacrylate copolymer fiber sheet as an immunoisolation membrane, cross-linked gelatin beads as the three-dimensional cell support, and a PDMS bag capable of changing its volume by supplying air from the outside as the oxygen supply mechanism, and tightly enclosing a three-dimensional cell support and an oxygen supply mechanism was produced using SIBS as a biocompatible adhesive.

First, an immunoisolation membrane laminated with a polymethacrylic acid-polymethacrylate copolymer fiber sheet was formed into a bag shape by adhesion using SIBS, and the crosslinked gelatin beads held in a net-like bag and a PDMS bag were introduced therein, and finally, opening of the back-like immunoisolation membrane was folded and physically sealed, so that an immunoisolation device in which the three-dimensional cell support and the oxygen supply mechanism were tightly enclosed inside was obtained.

### (Example 4)

The method of cell introduction in the immunoisolation device was examined.

A cell suspension of parenchymal hepatocytes was introduced into an immunoisolation device in which the three-dimensional cell support was previously tightly enclosed, and then an inlet was sealed (post-seeding method). On the other hand, the three-dimensional cell support to which parenchymal hepatocytes were previously adhered was introduced into the immunoisolation device, and the inlet was sealed and tightly enclosed (pre-seeding method). After incubation for 2 hours, when the three-dimensional cell support was taken out and the cells were observed, the cells were supported by the three-dimensional cell support in both the post-seeding method and the pre-seeding method. In particular, in the pre-seeding method, the distribution of cells was more uniform, and the proportion of cells fixed on the three-dimensional cell support was also higher.

Next, the retention rate of cells in vitro was examined by incubating the cell-containing immunoisolation device produced by the pre-seeding method in a medium for 2 days. As a result, the cell viability after incubation for 2 days was a sufficiently high value of 90%. In addition, it was confirmed that the medium in the cell-containing immunoisolation device was replaced with a Hanks solution by immersing the cell-containing immunoisolation device in the Hanks solution.

### (Comparative Example 1)

An immunoisolation device without a three-dimensional cell support was produced and its performance was examined. Specifically, an immunoisolation device was produced in the same manner as in Example 2 except that the three-dimensional cell support was not contained. The cell suspension of parenchymal hepatocytes was introduced into the immunoisolation device, the inlet was sealed, and the cells were incubated in a medium for 2 days.

As a result, the cells were unevenly aggregated toward a lower part of the device in two days, and the cell viability was as low as 20%.

### Reference Signs List

- 1: immunoisolation device
- 10: immunoisolation membrane
- 11: porous membrane
- 12: hydrophilic compound
- 13: covalent bond
- 20: oxygen supply mechanism
- 21: oxygen permeable membrane
- 22, 22a, 22b: flow path
- 30: three-dimensional cell support
- 40: cell
- S: solution
- S1: culture medium
- S2: solution

All publications, patents, and patent applications cited herein are hereby incorporated by reference in their entirety.

## Claims

1. An immunoisolation device for transplantation, comprising:
an immunoisolation membrane; and an oxygen supply mechanism and a three-dimensional cell support which are tightly enclosed inside the immunoisolation membrane by physical sealing, a biocompatible adhesive, or a combination thereof, in which
the immunoisolation membrane is a porous membrane provided with a hydrophilic layer on the outer surface, and
the oxygen supply mechanism can supply oxygen to a cell supported by the three-dimensional cell support through an oxygen permeable membrane.

2. The immunoisolation device according to claim 1, wherein the porous membrane is a fluororesin porous membrane.

3. The immunoisolation device according to claim 2, wherein the hydrophilic layer is a layer containing a hydrophilic compound physically adsorbed or fixed on the surface of the porous membrane.

4. The immunoisolation device according to claim 2, wherein the hydrophilic layer is a layer containing a hydrophilic compound introduced on the surface of the porous membrane via a covalent bond.

5. The immunoisolation device according to claim 3, wherein the hydrophilic compound is one or more selected from the group consisting of polyoxyethylene sorbitan fatty acid esters, collagen, gelatin, cellulose, agarose, alginic acid, chitosan, hydrophilic nylon, polyvinyl alcohol, polymethacrylic acid, polyethylene glycol, poly(2-methoxyethyl acrylate), and a 2-methacryloyloxyethyl phosphorylcholine polymer, and a derivative thereof.

6. The immunoisolation device according to claim 4, wherein the hydrophilic compound is one or more selected from the group consisting of polyethylene glycol, poly(2-methoxyethyl acrylate), a 2-methacryloyloxyethyl phosphorylcholine polymer, polyvinyl alcohol, polymethacrylic acid, polyacrylic acid, agarose, alginic acid, a polypeptide, and a saccharide, and a derivative thereof.

7. The immunoisolation device according to claim 1, wherein the three-dimensional cell support has a bead, fiber, mesh, nonwoven fabric, or sponge form.

8. The immunoisolation device according to claim 7, wherein the three-dimensional cell support contains one or more selected from the group consisting of cellulose, cross-linked agarose, chitosan, cross-linked gelatin, polyethylene, polypropylene, polystyrene, polycarbonate, polyester, polysulfone, polylactic acid, a silicone resin, a polyisobutylene-containing rubber, a styrene-isobutylene block copolymer, a styrene-isobutylene-styrene block copolymer, a fluororesin, polyvinylidene fluoride, silica, titanium, and a polyethylene glycol gel, and a derivative thereof.

9. The immunoisolation device according to claim 1, wherein the oxygen permeable membrane contains polydimethylsiloxane or a copolymer thereof.

10. The immunoisolation device according to claim 1, wherein the biocompatible adhesive is a fluorine-containing adhesive, a polyethylene glycol-containing adhesive, a cyanoacrylate-based adhesive, or an adhesive containing any one selected from the group consisting of polydimethylsiloxane, a styrene-isobutylene block copolymer, a styrene-isobutylene-styrene block copolymer, nylon, polyethylene terephthalate, polypropylene, and polycarbonate, and a derivative thereof.

11. A cell-containing immunoisolation device, in which a cell is introduced into the three-dimensional cell support in the immunoisolation device according to claim 1, and a solution in which the cell can survive is contained inside the immunoisolation membrane.

12. A method for exchanging a solution in the cell-containing immunoisolation device according to claim 11, comprising a step of immersing the cell-containing immunoisolation device in a solution different from the solution contained inside the immunoisolation membrane.

13. A method for transplanting the cell-containing immunoisolation device according to claim 11 into a non-human animal.
